(19) Europäisches Patentamt / European Patent Office / Office européen des brevets

(11) **EP 2 611 363 B1**

(12) **FASCICULE DE BREVET EUROPEEN**

(45) Date de publication et mention de la délivrance du brevet:
**13.09.2017 Bulletin 2017/37**

(21) Numéro de dépôt: **11761671.4**

(22) Date de dépôt: **25.08.2011**

(51) Int Cl.:
*A61B 6/03* $^{(2006.01)}$     *A61B 6/02* $^{(2006.01)}$
*G01N 23/04* $^{(2006.01)}$     *G01N 23/20* $^{(2006.01)}$
*G06T 11/00* $^{(2006.01)}$     *A61B 6/00* $^{(2006.01)}$

(86) Numéro de dépôt international:
**PCT/FR2011/051967**

(87) Numéro de publication internationale:
**WO 2012/028806 (08.03.2012 Gazette 2012/10)**

(54) **PROCEDE DE TRAITEMENT DE SPECTRES DE RAYONNEMENTS DIFFUSES PAR UN MATERIAU POUR L'OBTENTION D'UN SPECTRE DE RAYONNEMENT DIFFUSE PREMIER PAR LEDIT MATERIAU, DISPOSITIF ET PROGRAMME D'ORDINATEUR ASSOCIE**

VERFAHREN ZUR VERARBEITUNG VON SPEKTREN EINER VON EINEM MATERIAL GESTREUTEN STRAHLUNG FÜR DEN ERHALT EINES SPEKTRUMS EINER VON DIESEM MATERIAL EINFACH GESTREUTEN STRAHLUNG, VORRICHTUNG UND ZUGEHÖRIGES COMPUTERPROGRAMM

METHOD FOR PROCESSING SPECTRA OF RADIATION SCATTERED BY A MATERIAL IN ORDER TO OBTAIN A SPECTRUM OF RADIATION SINGLE SCATTERED BY SAID MATERIAL, DEVICE AND ASSOCIATED COMPUTER PROGRAM

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **31.08.2010 FR 1056912**

(43) Date de publication de la demande:
**10.07.2013 Bulletin 2013/28**

(73) Titulaire: **Commissariat à l'Énergie Atomique et aux Énergies Alternatives 75015 Paris (FR)**

(72) Inventeurs:
• **PAULUS, Caroline**
  **F-38000 Grenoble (FR)**
• **RINKEL, Jean**
  **F-38600 Fontaine (FR)**
• **TABARY, Joachim**
  **F-38100 Grenoble (FR)**

(74) Mandataire: **Santarelli**
  **49, avenue des Champs-Elysées**
  **75008 Paris (FR)**

(56) Documents cités:
**EP-A1- 1 566 771     US-A1- 2009 092 307**

**Description**

L'invention porte sur un procédé de traitement de spectres de rayonnement diffusé par un matériau et plus particulièrement sur un procédé d'extraction d'un spectre de rayonnement diffusé premier d'un ensemble d'au moins deux spectres mesurés de rayonnement diffusé par un matériau exposé à un rayonnement incident. Elle porte également sur un dispositif associé et sur un programme d'ordinateur pour un tel procédé d'extraction.

Le domaine d'application de l'invention s'étend en tout premier lieu à la spectrométrie d'un rayonnement diffusé X ou gamma, notamment mise en oeuvre dans l'analyse des matériaux. Une telle spectrométrie peut être utilisée pour la détection des explosifs.

La spectrométrie de diffusion de rayons X est basée sur l'exposition d'un matériau à un rayonnement X incident d'énergie de quelques dizaines à quelques centaines de keV. Les photons X, quand ils rencontrent le matériau sur lequel ils sont projetés, induisent différents types d'interactions avec celui-ci : fluorescence ou conversion interne (effet photoélectrique au cours duquel le photon transfert toute son énergie au matériau qui la restitue ensuite), diffusion inélastique (ou effet Compton qui inclut un changement de la direction du photon ainsi qu'une diminution de son énergie), création de paires positon - électron (uniquement pour des rayons X de très grande énergie non considérés dans la présente invention), ou diffusion Rayleigh (ou diffusion élastique, minoritaire aux énergies considérées).

L'invention utilise des sources de rayonnement électromagnétique dont l'énergie est comprise entre 0 et quelques centaines de keV, par exemple 300 keV. Il peut notamment s'agir de tubes générateurs de rayons X. Au delà de 30 keV, pour des matériaux organiques, le phénomène de diffusion Compton est prédominant.

Certaines caractéristiques des matériaux étudiés (coefficient d'atténuation linéaire $\mu$ (E), densité, rapport $\dfrac{Z}{A}$ entre le numéro atomique Z et le nombre de masse atomique A) peuvent en principe être déterminées sur la base de connaissances théoriques et de l'obtention du spectre diffusé premier du matériau exposé à un rayonnement X, c'est-à-dire le spectre de rayonnement diffusé qui serait obtenu dans une situation où chaque photon n'interagirait qu'une seule fois avec le matériau.

Or, les spectres de diffusion X comportent une composante importante de photons diffusés ayant interagi plusieurs fois avec le matériau. Cette composante est appelée le spectre de rayonnement diffusé multiple.

Certaines informations, notamment la densité du matériau, peuvent être obtenues sur la base du spectre de rayonnement diffusé total car le caractère atténuant du matériau affecte de la même manière les deux composantes, première et multiple, du spectre de rayonnement diffusé. Pour l'obtention d'une meilleure estimation de cette densité, ainsi que d'autres informations physico-chimiques, on constate que l'utilisation du spectre de rayonnement diffusé total mène à des résultats imparfaits.

Le document WO2007/007247 enseigne d'utiliser des données de transmission pour déterminer la composante de rayonnement diffusé multiple.

L'invention se place dans un autre contexte, puisqu'elle s'applique en premier lieu à un système d'analyse avec une source de rayonnement fortement collimatée, et un détecteur placé de telle sorte qu'il collecte un rayonnement diffusé.

Ce détecteur peut être placé dans le même demi-espace que la source vis-à-vis de la surface de matériau étudié, et également fortement collimaté. On parle alors d'une configuration en rétrodiffusion.

Le détecteur peut également être placé de telle sorte que le matériau étudié se situe entre la source et le détecteur. On parle alors d'une configuration en diffusion. Dans ce dernier cas, la collimation de la source et/ou du détecteur permet d'éviter que le rayonnement transmis par le matériau (i.e. n'ayant pas interagi avec le matériau) ne soit détecté. Le document EP-A1-1566771 divulgue un procédé de traitement de spectres de rayonnements diffusés par un matériau et l'obtention d'un spectre de rayonnement diffusé premier par ledit matériau. Le document US 6 320 933 porte sur une analyse de la diffusion de rayons X rétrodiffusés. Le rapport des intensités rétrodiffusées mesuré par un détecteur détectant toutes les radiations et un détecteur ne mesurant que la composante de rayonnement diffusé multiple donne uniquement une estimation de la densité. Au contraire, l'invention décrite ci-après, grâce à un algorithme de traitement particulier, permet la séparation entre rayonnement diffusé multiple et rayonnement diffusé primaire et l'obtention d'informations physico-chimiques plus nombreuses.

L'invention a pour objet de remédier, dans le contexte de cet agencement analytique, au problème évoqué ci-dessus, en proposant un procédé de correction du spectre de rayonnement diffusé total pour en extraire le rayonnement diffusé primaire.

Pour ce faire, il est proposé un procédé traitement de spectres de rayonnements diffusés par un matériau pour l'obtention d'un spectre de rayonnement diffusé premier par ledit matériau, dans lequel on expose le matériau à un faisceau d'irradiation incident émis par une source de rayonnement et on mesure au moins un spectre de rayonnement diffusé par le matériau. Le procédé selon l'invention est caractérisé par les étapes de procédé présentes dans la partie caractérisante de la revendication 1. L'invention porte également sur un dispositif de traitement de spectres de rayonnements diffusés pour l'obtention d'un spectre de rayonnement diffusé premier par un matériau, comprenant des moyens aptes à mettre en oeuvre le procédé selon l'invention. En particulier, l'invention s'étend à un dispositif de traitement de spectres de rayonnements diffusés pour l'obtention d'un spectre de rayonnement diffusé premier par un matériau, comprenant une source de rayonnement apte à émettre un faisceau d'irradiation incident vers le matériau, et au moins un détecteur apte à mesurer un spectre de rayonnement diffusé par le matériau. Le dispositif selon l'invention est caractérisé par les caractéristiques présentes dans la partie caractérisante de la revendication 9. A noter que les détecteur principal et secondaire(s) peuvent être réalisés par des appareils distincts. En variante, au moins deux des détecteurs sont réalisés par un seul et même appareil, qui est déplacé au cours du procédé selon l'invention, entre deux mesures de spectres.

Avantageusement, préalablement à la décomposition de la matrice des mesures, la matrice des poids est initialisée par extrapolation de données préétablies pour une pluralité de matériaux de référence. De préférence, l'extrapolation est faite en utilisant essentiellement comme variable d'extrapolation une estimation de la densité.

De plus, la matrice des spectres (S) est initialisée avec des spectres de rayonnement diffusé premier et multiple simulés pour un ou plusieurs matériaux de référence exposés audit faisceau d'irradiation de la source.

L'étape de décomposition de la matrice des mesures en matrices non négatives peut comprendre un processus itératif, chaque itération comprenant une mise à jour des coefficients desdites matrices non négatives. Ledit processus itératif est poursuivi jusqu'à la satisfaction d'un critère de convergence.

Selon un autre aspect du procédé selon l'invention, au moins un des spectres secondaires est avantageusement un spectre de rayonnement diffusé uniquement multiple. Autrement dit, ce spectre secondaire ne comporte pas de rayonnement diffusé premier, ou en quantité négligeable. En d'autres termes, avantageusement et selon l'invention, au moins un détecteur secondaire est agencé de sorte que son champ d'observation n'intersecte pas le faisceau d'irradiation au sein du matériau.

Le dispositif peut comprendre de plus au moins deux détecteurs collimatés avec un angle solide d'observation identique. Il peut comprendre au moins deux détecteurs collimatés et une source de rayonnement incident collimatée, les angles solides du faisceau d'irradiation et du champ d'observation dudit détecteur étant de préférence identiques. Dans le cas particulier d'un dispositif dont la source et le détecteur principal sont collimatés, le champ d'observation du détecteur est alors avantageusement de même dimension que le faisceau d'irradiation de la source au lieu de leur intersection.

L'invention s'étend également à un programme d'ordinateur comprenant une suite d'instructions apte, lorsqu'elle est exécutée par un microprocesseur, à mettre en oeuvre un procédé selon l'invention.

L'invention s'étend de plus à un procédé et un dispositif caractérisé en combinaison par tout ou partie des caractéristiques décrites ci-avant et ci-après

L'invention va maintenant être décrite en relation avec les figures annexées à titre d'illustrations non limitatives.

La figure 1 représente un agencement mis en oeuvre dans le cadre de l'invention.

La figure 2 présente le même agencement vu sous un autre angle.

La figure 3 présente les spectres mesurés par les détecteurs de l'agencement précédent, et leur décomposition entre rayonnement diffusé primaire et rayonnement diffusé multiple.

La figure 4 présente la part des composantes de rayonnement diffusé primaire et de rayonnement diffusé multiple dans le spectre de rayonnement diffusé total enregistré par un détecteur, en fonction de la position de celui-ci dans l'agencement des figures 1 et 2.

La figure 5 présente un spectre de rayonnement diffusé primaire servant à l'initialisation de données utilisées lors d'un procédé selon l'invention.

La figure 6 présente un spectre de rayonnement diffusé multiple servant à l'initialisation de données utilisées lors d'un procédé selon l'invention.

La figure 7 présente la variation du rapport entre quantité de photons de rayonnement diffusé primaire et quantité de photons de rayonnement diffusé multiple, en fonction de la densité du matériau et de la profondeur d'inspection, pour le premier détecteur de l'agencement des figures 1 et 2.

La figure 8 présente la même variation du rapport entre la quantité de photons du rayonnement diffusé primaire et la quantité de photons du rayonnement diffusé multiple, pour le deuxième détecteur de l'agencement des figures 1 et 2.

La figure 9 présente un agencement mettant en oeuvre l'invention pour l'analyse d'un volume uniforme de matériau.

La figure 10 présente les spectres utilisés lors de l'initialisation de données pour la mise en oeuvre du procédé selon l'invention dans le cadre de l'analyse du volume de matériau de la figure 8.

La figure 11 présente des données utilisées pour la mise en oeuvre du procédé selon l'invention dans le cadre de l'analyse du volume de matériau de la figure 9.

La figure 12 présente des résultats obtenus lors de l'analyse du volume de matériau de la figure 9.

La figure 13 présente un agencement mettant en oeuvre l'invention pour l'analyse d'un volume de matériau recouvert d'une couche extérieure d'un autre matériau.

La figure 14 présente les spectres utilisés lors de l'initialisation de données pour la mise en oeuvre du procédé selon l'invention dans le cadre de l'analyse du volume de matériau de la figure 13.

La figure 15 présente des données utilisées pour la mise en oeuvre du procédé selon l'invention dans le cadre de l'analyse du volume de matériau de la figure 13.

La figure 16 présente des résultats obtenus lors de l'analyse du volume de matériau de la figure 13.

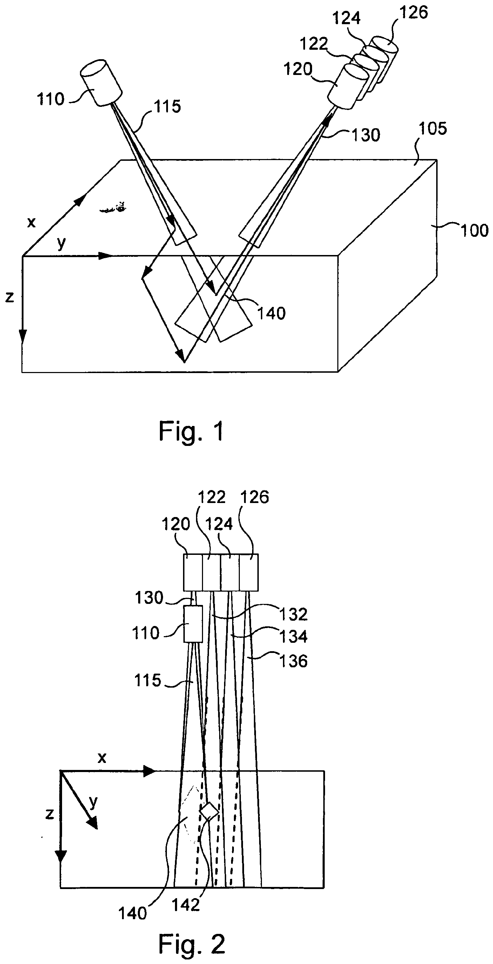

En figures 1 et 2 on a représenté un agencement mettant en oeuvre l'invention. Un volume parallélépipédique de matériau 100 à analyser est représenté. Ce volume de matériau 100 présente une surface 105 plane définie par des axes x et y perpendiculaires l'un à l'autre, et un axe de profondeur z perpendiculaire à la surface 105.

Une source 110 de rayonnement X est dirigée vers la surface 105, et génère un faisceau 115 de rayons X, dit faisceau d'irradiation. La source 110 est collimatée, et le faisceau 115 a donc un angle solide d'ouverture limité. Le faisceau d'irradiation 115 a ici un axe central disposé dans un plan perpendiculaire à la surface 105, et est incliné par rapport à celle-ci dans ce plan. Du fait de la collimation de la source, le faisceau d'irradiation est spatialement délimité autour d'un axe central, dit axe du faisceau d'irradiation. Ce faisceau d'irradiation, pouvant également être appelé champ d'irradiation, peut être conique (on parle alors de cône d'irradiation), pyramidal, ou d'autres formes, selon la géométrie du collimateur.

Quatre détecteurs de rayonnement, dits premier, deuxième, troisième et quatrième détecteur, et respectivement désignés par les références 120, 122, 124 et 126 sont placés face à la surface 105. Ils sont ainsi tous les quatre disposés pour mesurer un rayonnement rétrodiffusé par le volume de matériau 100. Ils sont tous les quatre collimatés. Ainsi, à chaque détecteur correspond un champ d'observation, ce dernier correspondant à l'angle solide d'observation défini par le collimateur. Ce champ d'observation est spatialement délimité autour d'un axe central, dit axe du champ d'observation. Chaque axe central définit un angle donné par rapport à la surface 105. Le champ d'observation, pouvant également être appelé champ de détection, peut être conique (on parle alors de cône d'observation), pyramidal, ou d'autres formes, selon la géométrie du collimateur.

Dans le mode de réalisation présenté, chaque détecteur a un angle solide d'observation de la surface 105 identique à l'angle solide d'ouverture de la source 110. Le champ d'observation du premier détecteur 120 est référencé 130 et a un axe central qui est dans le même plan que l'axe central du faisceau, faisceau d'irradiation 115 émis par la source. L'intersection du champ d'observation 130 du premier détecteur 120 et du faisceau d'irradiation 115 de la source 110 définit un volume, dit volume d'inspection 140. En effet, comme on le verra plus tard, le détecteur 120 réalise l'inspection du volume 140 du matériau 100. Selon les positions et orientations respectives de la source 115 et du premier détecteur 120, le volume d'inspection 140 est situé à une certaine profondeur sous la surface 105 du matériau analysé, dite profondeur d'inspection.

Dans le mode de réalisation présenté, tous les détecteurs, ainsi que la source, sont disposés, suivant l'axe central de leur champ d'observation ou faisceau d'irradiation, à une même distance de la surface du matériau.

Les champs d'observation des deuxième, troisième et quatrième détecteurs 122, 124 et 126 sont référencés 132, 134 et 136 et ont des axes centraux qui sont ici parallèles à l'axe central du champ d'observation du détecteur 120. Ces quatre axes sont de plus coplanaires dans le mode de réalisation présenté. L'axe central du champ d'observation 132 rencontre la surface 105 à une distance de 0,5 cm du point où l'axe du champ d'observation 130 rencontre cette surface. Les axes centraux des champs d'observation 134 et 136 font de même à des distances respectivement de 1 cm et 1,5 cm. Par ailleurs, chacun des détecteurs est à la même distance de la surface 105, ce qui a pour conséquence que les surfaces d'intersection entre les champs d'observation de ces détecteurs et la surface 105 sont identiques en forme et en dimension.

Le champ d'observation 132 a ici un volume d'intersection 142 avec le faisceau 115 dans la masse du volume de matériau 100. Ce volume 142 est de volume inférieur au volume 140 précédemment défini. Par contre, les champs d'observation 134 et 136 ne rencontrent pas le faisceau 115 dans le matériau 100. Ainsi, le rayonnement détecté par les troisième et quatrième détecteurs 124 et 126 ne comportent pas de rayonnement diffusé primaire. Par contre, le rayonnement détecté par les premier et deuxième détecteurs 120 et 122 comportent une part de rayonnement diffusé primaire, cette part étant plus importante pour le détecteur 120 que pour le détecteur 122.

Selon l'invention, il est essentiel qu'au moins un détecteur soit tel que son champ d'observation intersecte le faisceau d'irradiation de la source dans le matériau analysé. Ainsi, le rayonnement détecté par ce détecteur comprend une part de rayonnement diffusé primaire.

Il est également avantageux qu'au moins un détecteur soit tel que son champ d'observation n'intersecte pas le faisceau d'irradiation de la source dans ledit matériau. Le rayonnement détecté par ce détecteur ne comprend pas de rayonnement diffusé primaire, ce qui permet une extraction rapide et précise du spectre de rayonnement diffusé primaire de l'ensemble des données collectées.

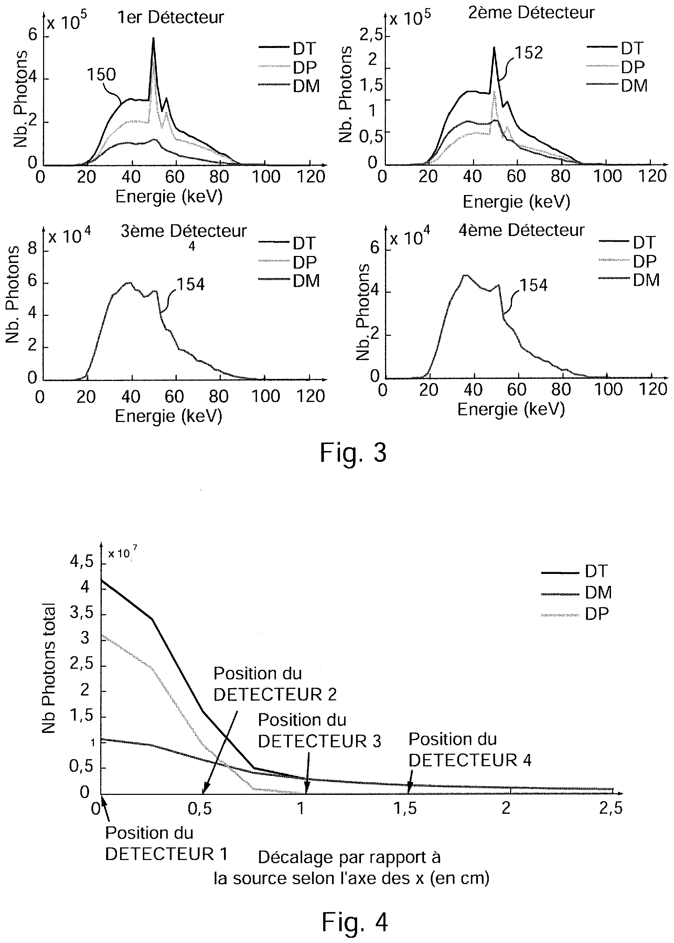

En référence à la figure 3, on a représenté les spectres 150, 152, 154 et 156 mesurés par les premier, deuxième, troisième et quatrième détecteurs, respectivement, de l'agencement des figures 1 et 2, pour un matériau étalon. Les spectres 150 et 152 comporte une forte composante en diffusé premier, visible notamment par la présence d'un pic à 50 keV. Les deux composantes estimées pour chacun de ces deux spectres sont représentées : la composante en diffusé premier est proportionnellement plus importante pour le spectre 150 que pour le spectre 152. Les spectres 154 et 156, d'intensité globale plus faible, n'ont quant eux aucune composante de rayonnement diffusé premier.

La figure 4, qui est un résultat de simulation, présente en ordonnées l'intensité des différentes composantes de rayonnement diffusé (primaire DP, multiple DM et total DT) présentes dans les signaux recueillis par les détecteurs 120, 122, 124 et 126. L'axe des abscisses représente la distance entre le point d'intersection de l'axe central du champ d'observation du premier détecteur avec la surface 105 et les points d'intersection des axes centraux des champs d'observation des détecteurs suivants avec cette même surface 105.

Le détecteur 120 collecte un nombre de photons total de l'ordre de 4,2 107, pour une durée d'acquisition de 2 minutes, qui se décompose en une fraction majoritaire de rayonnement diffusé premier, proche de 3,1 107, et une fraction secondaire de rayonnement diffusé multiple, proche de 1,1 107. Le détecteur 122 collecte quant à lui 1,6 107 photons dont 0,9 107 constituent un signal de rayonnement diffusé primaire, et 0,7 107 un signal de rayonnement diffusé multiple. Les détecteurs 124 et 126 collectent quant à eux des signaux plus faibles, constitués entièrement de rayonnement diffusé multiple.

Selon le procédé, on constitue une matrice X comprenant les différents spectres mesurés par les détecteurs, rangés en lignes. La matrice X s'écrit $X = (X_1, X_2, X_3, X_4)^T$, si $X_i$ désigne le spectre obtenu par le détecteur i et T l'opération de transposition matricielle. D'une façon générale, si on note de plus Nd le nombre de détecteurs la matrice X s'écrit $X = (X_1, ..., X_{Nd})^T$.

Les détecteurs collectent les photons sur des canaux discrets, dont on note le nombre Ne, pour « nombre de canaux en énergie ». La matrice X s'écrit alors

$$X = \begin{bmatrix} x_{1,1} & \cdots & x_{1,Ne} \\ \cdots & \cdots & \cdots \\ x_{Nd,1} & & x_{Nd,Ne} \end{bmatrix}.$$

Chaque terme $x_{ik}$ de la matrice X, correspond selon cette notation à une quantité de photons d'énergie k mesurée par le détecteur i.

On décompose la matrice X, en deux matrices non négatives A et S dites matrices des poids et des spectres, dont l'initialisation est décrite ultérieurement. La décomposition a pour objectif de définir deux matrices non négatives A et S, telles que $X \approx A.S$. Par matrice non négative (ou matrice positive), on entend une matrice dont tous les termes sont positifs ou nuls.

La matrice A des poids est, dans notre exemple, une matrice à deux colonnes et à quatre lignes, dont les termes représentent respectivement des poids du spectre de rayonnement diffusé premier et des poids du spectre de rayonnement diffusé multiple sur les quatre détecteurs.

D'une façon générale, la matrice A des poids a un nombre de lignes égal au nombre de détecteurs, c'est-à-dire Nd. Le nombre de colonnes de la matrice A correspond au nombre de spectres diffusés que l'on souhaite extraire, en l'occurrence deux : un spectre diffusé premier et un spectre diffusé multiple. Ainsi, chaque élément de la matrice A est un poids estimé d'une des composantes de rayonnement diffusé dans le signal mesuré par l'un des détecteurs. Chaque terme $a_{ij}$ de la matrice A est la proportion du spectre diffusé j (spectre diffusé premier ou spectre diffusé multiple) mesuré par le détecteur i.

La matrice S des spectres est une matrice à deux lignes, représentant chacune un spectre estimé, soit de rayonnement diffusé premier, soit de rayonnement diffusé multiple. La matrice S a par ailleurs un nombre de colonnes égal au nombre de canaux d'énergie, soit Ne, chaque spectre étant discrétisé sur les Ne canaux. Chaque terme $s_{jk}$ de la matrice S est donc une quantité de photons du spectre j d'énergie comprise dans la plage d'énergie Ek correspondant au canal k.

Comme précédemment indiqué, les matrices A et S sont définies telles que $X \approx A.S$. Le problème revient alors à déterminer les matrices A et S telles qu'une distance entre X et le produit AS soit minimum, avec A et S ne comprenant que des termes positifs ou nuls. La distance peut être exprimée par la fonctionnelle $\|X-AS\|^2$, représentant une distance Euclidienne.

Pour minimiser cette fonctionnelle, plusieurs méthodes sont envisageables comme par exemple une descente de gradient classique, sous la contrainte que A et S soient positives (ou non négatives). Pour assurer un bon compromis entre rapidité de convergence et facilité d'implémentation, Lee et Seung ont proposé des lois de mise à jour multiplicatives, comme décrit dans la publication Lee, D. & Seung, H.S., 2001. Algorithms for Non-negative Matrix Factorization. Adv. Neural Info. Proc. Syst., (13), 556-562.

Ces lois de mise à jour, appliquées selon un processus itératif, sont les suivantes

$a_{i,j}$ est remplacé, à chaque itération, par

$$a_{i,j} \frac{(XS^T)_{i,j}}{(ASS^T)_{i,j}},$$

puis $s_{j,k}$ est remplacé, à chaque itération, par

$$s_{j,k} \frac{(A^T X)_{j,k}}{(A^T AS)_{j,k}},$$

où pour une matrice donnée, les indices i et j font référence à l'élément de la matrice à la ligne i et la colonne j et où T est le symbole de la transposition.

Ces lois de mise à jour assurent que la fonctionnelle précédemment définie décroît au fur et à mesure des itérations et converge vers un minimum local.

Le nombre d'itération peut être fixé arbitrairement, ou déterminé selon un critère de convergence. Un critère de convergence peut être un seuil $\varepsilon$, le processus itératif étant stoppé lorsque $\|X\text{-}AS\|^2 \le \varepsilon$.

Préalablement à la décomposition de la matrice X, il est nécessaire d'initialiser les matrices des poids A et des spectres S utilisées lors de la première itération. Naturellement, cette initialisation doit respecter les contraintes de positivité.

Ces matrices peuvent être initialisées arbitrairement, mais les inventeurs ont montré qu'il était préférable que les matrices A et S soient initialisées comme décrit ci-dessous.

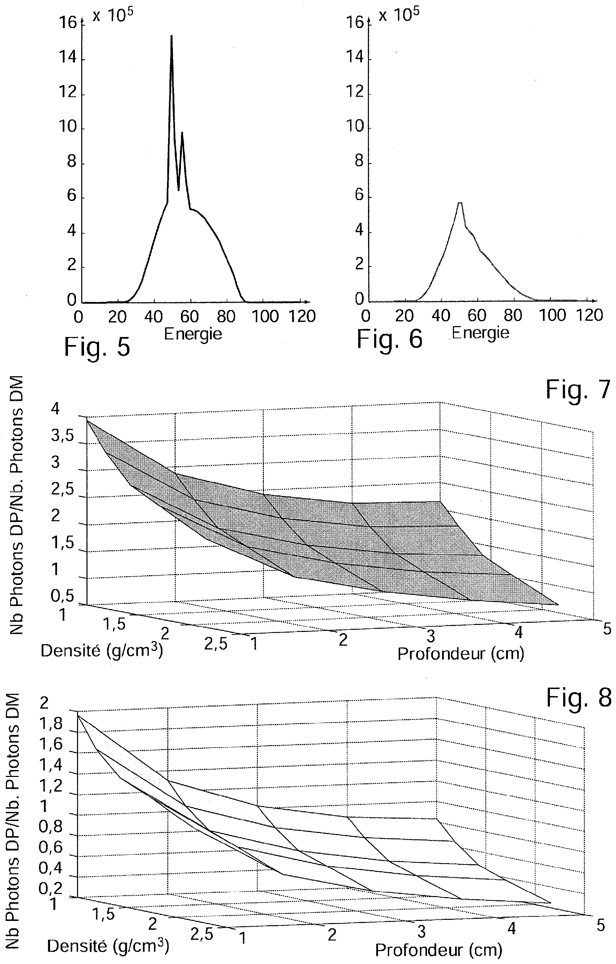

La matrice S est préférentiellement initialisée avec des spectres de rayonnement diffusé premier et multiple simulés pour un matériau donné. Un exemple est représenté en figure 5, pour le spectre de rayonnement diffusé premier, qui est utilisé pour la première ligne de la matrice S. Le matériau utilisé est de l'aluminium. La figure 6 présente le spectre de rayonnement diffusé multiple de l'aluminium, utilisé pour initialiser la deuxième ligne de la matrice S. Ce spectre simulé offre une allure, ou forme générale, constituant une estimation réaliste de la distribution en intensité sur les différents canaux en énergie du spectre correspondant de la matrice S. Précisons que la simulation de l'intensité exacte des canaux du spectre n'est pas un paramètre critique à ce stade, le plus important étant de disposer de valeurs relatives réalistes.

La matrice des poids A est initialisée en tenant compte tout d'abord du fait que certains des détecteurs (ici les troisième et quatrième détecteurs 124 et 126) ne peuvent pas recevoir de photons n'ayant subi qu'un seul changement de trajectoire dans le matériau, car leur champ d'observation et le faisceau d'irradiation n'ont pas d'intersection. Ainsi, le poids du spectre de rayonnement diffusé primaire est nul dans les spectres mesurés par ces détecteurs.

Puis, pour les détecteurs dont le positionnement leur permet d'observer une partie du spectre de rayonnement diffusé primaire, c'est-à-dire les détecteurs dont le champ d'observation a une intersection avec le faisceau d'irradiation dans la masse du matériau, le rapport entre intensité du spectre de rayonnement diffusé multiple et spectre de rayonnement diffusé primaire est déterminé sur la base de données préétablies et conservées dans une base de données, comprenant des simulations réalisées pour différents matériaux, et diverses profondeurs d'inspection.

Des simulations de transport de particules basées sur un protocole de type Monte-Carlo (Géant, MCNP) ou un autre protocole, notamment celui du logiciel SindBad utilisé dans le mode de réalisation présenté, permettent de connaître le rapport entre la quantité de photons relevant de la diffusion primaire et la quantité de photons relevant de la diffusion multiple.

Dans le mode de réalisation présenté, cinq matériaux ont été utilisés pour former une base de données. Ces matériaux sont l'eau ($H_2O$), le Plexiglas (marque déposée), le Delrin (marque déposée), le Kynar (marque déposée) ainsi que le Teflon (marque déposée). Les densités de ces matériaux varient entre 1 et 2,2. La base de données comprend des simulations effectuées pour cinq profondeurs d'inspection variant de 1 à 5 cm pour chacun des matériaux, l'agencement étant celui décrit en figure 1.

La figure 7 représente la nappe obtenue pour le premier détecteur de la figure 1 en représentant sur un axe horizontal la densité du matériau, sur un deuxième axe horizontal la profondeur d'inspection, et sur l'axe vertical le rapport entre des photons relevant du diffusé primaire et les photons relevant du diffusé multiple. On constate qu'un maximum est obtenu pour des faibles densités et une profondeur minimale. Ainsi, pour une densité de 1 et une profondeur de 1 cm le rapport s'approche alors de 4. A l'inverse, pour une profondeur de 5 cm et une densité de 2,5 le rapport est de 0,5 seulement.

En figure 8, on a représenté la nappe obtenue pour le deuxième détecteur. Des axes horizontaux représentent une nouvelle fois la densité et la profondeur en cm alors que l'axe vertical représente le rapport des nombres de photons relevant du diffusé primaire par rapport au nombre de photons relevant du diffusé multiple. Là aussi, les valeurs maximales de ce rapport sont obtenues pour des faibles densités et une faible profondeur. Ainsi, le rapport pour une densité de 1 et une profondeur de 1 cm avoisine la valeur de 2. A l'inverse, pour une profondeur de 5 cm et une densité de 2,5 le rapport n'est plus que de 0,3.

La matrice A est initialisée en donnant arbitrairement une valeur de 1 à tous les poids du spectre de rayonnement diffusé multiple. Les poids non nuls du spectre de rayonnement diffusé primaire sont initialisés en lisant sur la nappe correspondant au détecteur (en figure 7 ou en figure 8), la valeur du rapport entre la quantité de photons relevant du diffusé primaire par rapport à la quantité de photons relevant du diffusé multiple, la nappe pouvant être approchée par un polynôme, par exemple un polynôme de degré 3, au sens des moindres carrés.

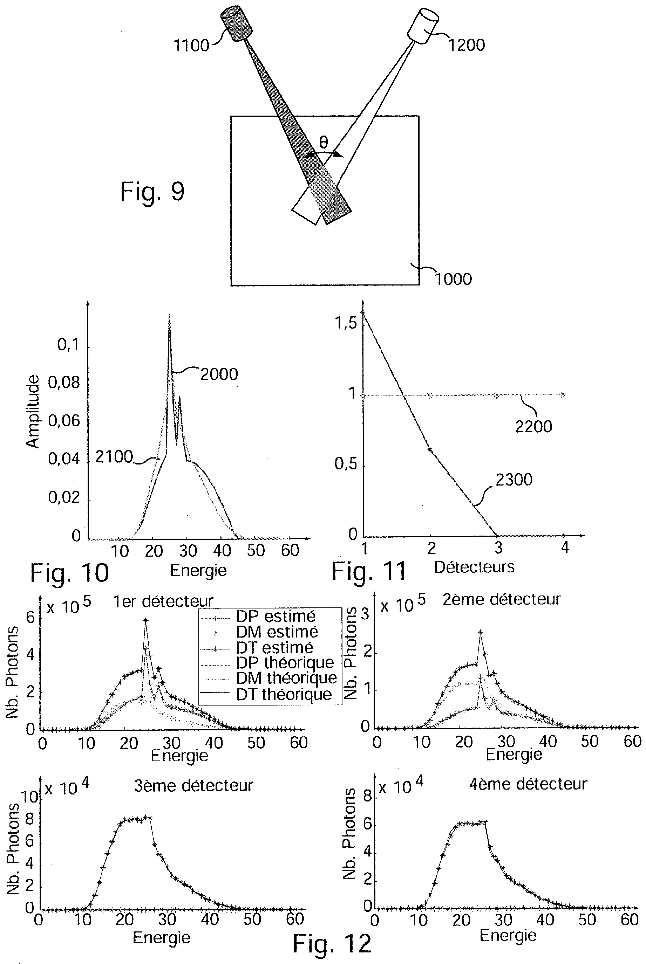

En figure 9 on a représenté le dispositif expérimental utilisé pour l'étude d'un bloc homogène de Delrin. Le volume de Delrin est référencé 1000 alors que la source de rayonnement X est référencée 1100, un détecteur 1200 étant également visible. Trois autres détecteurs, orientés parallèlement au premier détecteur 1200, sont non représentés. Dans ce mode de réalisation la source 1100 est à 23 cm de la surface du matériau, cette distance étant mesurée suivant l'axe central du faisceau de rayons X. L'angle d'ouverture de la source est de 2,4 degrés, le détecteur 1200 ainsi que les trois autres détecteurs non représentés sont des détecteurs au tellurure de cadmium. Les quatre détecteurs sont collimatés avec un angle d'observation de 2,4 degrés égal à l'angle du faisceau d'irradiation. L'angle entre l'axe central du faisceau d'irradiation et l'axe central des champs de détection du détecteur 1200 est de 120 degrés. La profondeur du volume d'inspection est de 4 cm, le volume 1000 étant un cube de 8 cm de côté.

Les matrices S et A sont initialisées avec les données représentées aux figures 10 et 11, respectivement. Ainsi, le spectre 2000 est utilisé pour l'initialisation du spectre de rayonnement diffusé primaire et le spectre 2100 est utilisé pour l'initialisation du spectre de rayonnement diffusé multiple. Les poids du spectre de rayonnement diffusé multiple dans les quatre détecteurs sont initialisés à 1 uniformément comme cela est représenté en référence 2200 en figure 10. Les poids du spectre de rayonnement diffusé primaire sont initialisés à environ 1,6 ; 0,7 ; 0 et 0 pour les premier, deuxième, troisième et quatrième détecteurs, respectivement, comme cela ressort de la courbe 2300 en figure 11.

La figure 12 présente les résultats obtenus à l'issue des 50 itérations pour chacun des quatre détecteurs. En haut à gauche de la figure 12 est représentée la décomposition du spectre de rayonnement diffusé total mesuré par le détecteur 1 (référence 1200, figure 9) en sa composante de rayonnement diffusé premier et sa composante de rayonnement diffusé multiple. Une présentation identique est effectuée en haut à droite de la figure 12 pour le spectre de rayonnement diffusé total mesuré par le deuxième détecteur de l'agencement. On note que la composante de rayonnement diffusé primaire est proportionnellement plus faible dans ce spectre que pour le spectre mesuré par le premier détecteur. Les graphiques du bas de la figure 12 présentent les spectres de rayonnement diffusé multiple mesurés par les troisième et quatrième détecteurs. Ils sont identiques, mis à part que l'intensité du dernier spectre est plus faible. Tous ces graphiques confrontent des données obtenues à l'issue du processus de factorisation à des données de simulation théorique. On constate sur chacun des graphiques que la concordance est excellente.

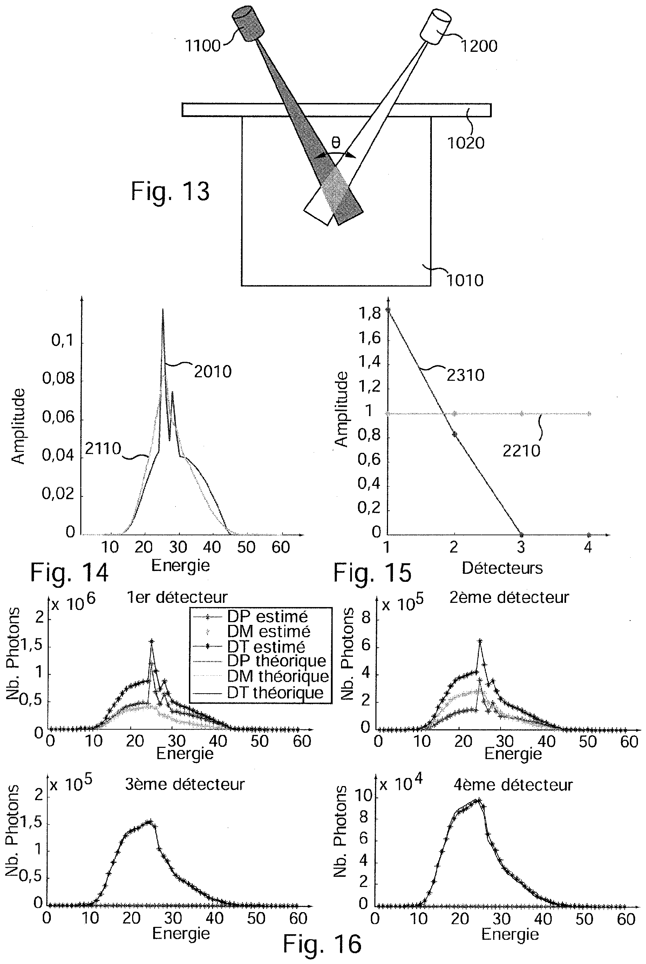

En figure 13 on a représenté l'agencement utilisé pour une étude menée sur un volume de Kynar surmonté d'une fine épaisseur de nylon, cette dernière pouvant représenter une surface extérieure de sacs ou de valises. Le volume de Kynar est référencé 1110 et la fine surface de nylon est référencée 1020. Les angles solides du faisceau d'irradiation et des détecteurs ainsi que l'angle de diffusion sont identiques à ceux présentés dans l'agencement de la figure 9. C'est également le cas des distances entre la source et le matériau. La profondeur d'inspection est cette fois-ci de 2 cm, le volume de Kynar étant un cube de 8 cm de côté et la couche de nylon ayant une épaisseur de 4 mm.

La matrice S est initialisée avec les données représentées en figure 14 qui sont composées d'une part d'un spectre de rayonnement diffusé primaire 2010 et d'autre part d'un spectre de rayonnement diffusé multiple 2110. La matrice A est initialisée avec les données représentées en figure 14 où les poids relatifs du spectre de rayonnement diffusé primaire des premier et deuxième détecteurs sont choisis à 1,85 et 0,8, comme cela apparaît en référence 2310.

La figure 16 présente les résultats obtenus après convergence de l'algorithme de factorisation, pour les quatre détecteurs. En haut à gauche on observe que pour le premier détecteur la composante de rayonnement diffusé de primaire est majoritaire dans le spectre. Alors que pour le spectre mesuré par le deuxième détecteur en haut à droite la composante de rayonnement diffusé multiple est plus importante. Les deux figures du bas représentent les spectres mesurés par les troisième et quatrième détecteurs, qui ne comportent aucune composante en diffusé primaire. Ces deux spectres sont identiques, mis à part que l'intensité du dernier spectre est plus faible. Les données obtenues par l'algorithme de factorisation sont confrontées à des données obtenues par des simulations sur la base de données théoriques. On constate que les données obtenues par le procédé selon l'invention et les données simulées se superposent d'excellente manière.

L'invention est mise en oeuvre, dans un mode de réalisation, par un programme informatique, par exemple stocké sur un support lisible par un ordinateur.

Dans le cadre de variantes, au lieu d'utiliser un faisceau d'irradiation dont la forme est celle d'un cône ou d'un autre volume, on peut utiliser un faisceau d'irradiation plat. Par ailleurs, les détecteurs peuvent être en nombre variable, avec des axes de champs d'observation qui ne sont pas nécessairement coplanaires. Ils peuvent aussi être disposés pour mesurer un rayonnement diffusé en avant, et non pas rétrodiffusé. De manière générale, l'invention ne se limite aux modes de réalisation précisément décrits et s'étend aux variantes à la portée de l'homme du métier.

## Revendications

1. Procédé de traitement de spectres de rayonnements diffusés par un matériau (100) pour l'obtention d'un spectre de rayonnement diffusé premier par ledit matériau, dans lequel on expose le matériau (100) à un faisceau d'irradiation (115) incident émis par une source (110) de rayonnement et on mesure au moins un spectre de rayonnement diffusé par le matériau, **caractérisé en ce que** :

   - on mesure un premier spectre (150) de rayonnement diffusé par le matériau (100) à l'aide d'un détecteur (120), dit détecteur principal, agencé de sorte que son champ d'observation (130) intersecte le faisceau d'irradiation (115) au sein du matériau (100),
   - on mesure au moins un autre spectre (152, 154, 156) de rayonnement diffusé par le matériau (100), dit spectre secondaire, à l'aide d'au moins un détecteur (122, 124, 126) dit détecteur secondaire,

- on constitue une matrice (X), dite matrice des mesures, à partir des spectres précédemment mesurés,
- puis on décompose ladite matrice des mesures en deux matrices non négatives, à savoir une matrice (A) dite matrice des poids, dont chaque terme $a_{ij}$ représente la proportion d'un rayonnement diffusé j premier ou multiple mesuré par le détecteur i et une matrice (S) dite matrice des spectres, cette dernière comprenant un spectre de rayonnement diffusé multiple estimé et un spectre de rayonnement diffusé primaire estimé, cette décomposition étant précédée d'une étape d'initialisation de la matrice des poids (A) et de la matrice des spectres (S).

2. Procédé selon la revendication 1, **caractérisé en ce qu'**au moins un détecteur secondaire (124) est agencé de sorte que son champ d'observation (134) n'intersecte pas le faisceau d'irradiation (115) au sein du matériau (100).

3. Procédé selon la revendication 1 ou la revendication 2, **caractérisé en ce que** la matrice des poids (A) est initialisée par extrapolation de données préétablies pour une pluralité de matériaux de référence (Fig. 7 et 8).

4. Procédé selon la revendication 3, **caractérisé en ce que** l'extrapolation est faite en utilisant essentiellement comme variable d'extrapolation une estimation de la densité.

5. Procédé selon l'une des revendications précédentes, **caractérisé en ce que** la matrice des spectres (S) est initialisée avec des spectres de rayonnement diffusé premier et multiple (Fig. 5 et 6) simulés pour un matériau de référence exposé audit faisceau d'irradiation (115).

6. Procédé selon l'une des revendications précédentes, **caractérisé en ce qu'**il comprend un processus itératif dont chaque pas comprend une telle décomposition en matrices non négatives.

7. Procédé selon la revendication précédente, **caractérisé en ce que** ledit processus itératif est poursuivi jusqu'à la satisfaction d'un critère de convergence portant sur une proximité entre la matrice des mesures (X) et un produit de matrices non négatives (AS).

8. Procédé selon l'une de revendications précédentes, **caractérisé en ce qu'**au moins deux des détecteurs (120, 122, 124, 126) utilisés sont collimatés et disposés, suivant l'axe central de leur champ d'observation, à une même distance d'une surface (105) du matériau exposée au faisceau d'irradiation (115).

9. Dispositif de traitement de spectres de rayonnements diffusés pour l'obtention d'un spectre de rayonnement diffusé premier par un matériau (100), comprenant une source (110) de rayonnement apte à émettre un faisceau d'irradiation (115) incident vers le matériau (100), et au moins un détecteur apte à mesurer un spectre de rayonnement diffusé par le matériau (100),
**caractérisé en ce qu'**il comprend :

- un premier détecteur (120), dit détecteur principal, apte à mesurer un premier spectre (150) de rayonnement diffusé par le matériau (100), et agencé de sorte que son champ d'observation (130) intersecte le faisceau d'irradiation (115) au sein du matériau (100),
- au moins un détecteur (122, 124, 126) dit détecteur secondaire, apte à mesurer au moins un autre spectre (152, 154, 156), dit spectre secondaire, de rayonnement diffusé par le matériau (100),
- des moyens de constitution d'une matrice (X) dite matrice des mesures, à partir des spectres mesurés,
- des moyens informatiques de décomposition de ladite matrice des mesures en deux matrices non négatives, à savoir une matrice (A) dite matrice des poids dont chaque terme $a_{ij}$ représente la proportion d'un rayonnement diffusé j premier ou multiple mesuré par le détecteur i et une matrice (S) dite matrice des spectres, cette dernière comprenant un spectre de rayonnement diffusé multiple estimé et un spectre de rayonnement diffusé primaire estimé ; ces moyens de décomposition étant aptes à exécuter une phase préalable d'initialisation de la matrice des poids (A) et de la matrice des spectres (S).

10. Dispositif selon la revendication 9, **caractérisé en ce qu'**au moins l'un des détecteurs secondaires (124) est agencé de sorte que son champ d'observation (134) n'intersecte pas le faisceau d'irradiation (115) au sein du matériau (100).

11. Dispositif selon l'une des revendications précédentes, **caractérisé en ce qu'**au moins deux des détecteurs (120, 122, 126, 128) sont collimatés avec un angle solide d'observation identique.

12. Dispositif selon l'une des revendications précédentes, **caractérisé en ce qu'**au moins l'un des détecteurs (120, 122, 124, 126) est collimaté et **en ce que** la source de rayonnement incident (110) est collimatée, les angles solides du faisceau d'irradiation et du champ d'observation dudit détecteur étant identiques.

13. Dispositif selon l'une des revendications précédentes, **caractérisé en ce que** la source (110) est une source de rayonnement X ou gamma.

14. Dispositif selon l'une des revendications précédentes, **caractérisé en ce que** les détecteurs (120, 122, 126, 128) principal et secondaires sont agencés pour mesurer un rayonnement rétrodiffusé.

15. Programme d'ordinateur comprenant une suite d'instructions apte, lorsqu'elle est exécutée par un microprocesseur, à mettre en oeuvre un procédé selon l'une des revendications 1 à 8.

**Patentansprüche**

1. Verfahren zur Verarbeitung von Spektren von durch ein Material (100) gestreuten Strahlungen zum Erhalt eines von dem Material einfach gestreuten Strahlungsspektrums, wobei das Material (100) einem einfallenden Strahlungsbündel (115) ausgesetzt wird, das von einer Strahlungsquelle (110) emittiert wird, und zumindest ein Spektrum der von dem Material gestreuten Strahlung gemessen wird, **dadurch gekennzeichnet, dass**:

   - ein erstes Spektrum (150) der von dem Material (100) gestreuten Strahlung mit Hilfe eines Sensors (120), Hauptsensor genannt, gemessen wird, der so angeordnet ist, dass sein Betrachtungsfeld (130) das Strahlungsbündel (115) innerhalb des Materials (100) schneidet,
   - zumindest ein weiteres Spektrum (152, 154, 156) der von dem Material (100) gestreuten Strahlung, sekundäres Spektrum genannt, mit Hilfe zumindest eines Sensors (122, 124, 126), Nebensensor genannt, gemessen wird,
   - eine Matrix (X), Messungsmatrix genannt, ausgehend von den zuvor gemessenen Spektren erstellt wird,
   - dann die Messungsmatrix in zwei nicht negative Matrizes zerlegt wird, nämlich in eine Matrix (A), Gewichtsmatrix genannt, von der jeder Term $a_{ij}$ den Anteil einer von dem Sensor i gemessenen, einfach oder mehrfach gestreuten Strahlung j darstellt, und in eine Matrix (S), Spektrenmatrix genannt, wobei letztere ein Spektrum geschätzter, mehrfach gestreuter Strahlung und ein Spektrum geschätzter, einfach gestreuter Strahlung umfasst, wobei dieser Zerlegung ein Schritt des Initialisierens der Gewichtsmatrix (A) und der Spektrenmatrix (S) vorangeht.

2. Verfahren nach Anspruch 1, **dadurch gekenn-**

**zeichnet, dass** zumindest ein Nebensensor (124) so angeordnet ist, dass sein Betrachtungsfeld (134) das Strahlungsbündel (115) innerhalb des Materials (100) nicht schneidet.

3. Verfahren nach Anspruch 1 oder Anspruch 2, **dadurch gekennzeichnet, dass** die Gewichtsmatrix (A) durch Extrapolation von vorerstellten Daten für eine Mehrzahl von Referenzmaterialien initialisiert wird (Fig. 7 und 8).

4. Verfahren nach Anspruch 3, **dadurch gekennzeichnet, dass** die Extrapolation erfolgt, indem im Wesentlichen als Variable bei der Extrapolation eine Schätzung der Dichte verwendet wird.

5. Verfahren nach einem der vorangehenden Ansprüche, **dadurch gekennzeichnet, dass** die Spektrenmatrix (S) mit Spektren einfach und mehrfach gestreuter Strahlung initialisiert wird (Fig. 5 und 6), die bei einem Referenzmaterial simuliert werden, das dem Strahlungsbündel (115) ausgesetzt wird.

6. Verfahren nach einem der vorangehenden Ansprüche, **dadurch gekennzeichnet, dass** es einen iterativen Prozess umfasst, bei dem jeder Schritt eine solche Zerlegung in nicht negative Matrizes umfasst.

7. Verfahren nach dem vorangehenden Anspruch, **dadurch gekennzeichnet, dass** der iterative Prozess bis zur Erfüllung eines Konvergenzkriteriums fortgesetzt wird, das eine Annäherung zwischen der Messungsmatrix (X) und einem Produkt nicht negativer Matrizes (AS) betrifft.

8. Verfahren nach einem der vorangehenden Ansprüche, **dadurch gekennzeichnet, dass** zumindest zwei der verwendeten Sensoren (120, 122, 124, 126) kollimiert und in einer Mittelachse ihres Betrachtungsfeldes in gleichem Abstand von einer Fläche (105) des dem Strahlungsbündel (115) ausgesetzten Materials angeordnet sind.

9. Vorrichtung zur Verarbeitung von Spektren gestreuter Strahlungen zum Erhalt eines von einem Material (100) einfach gestreuten Strahlungsspektrums, umfassend eine Strahlungsquelle (110), die dazu geeignet ist, ein einfallendes Strahlungsbündel (115) zum Material (100) hin zu emittieren, und zumindest einen Sensor, der dazu geeignet ist, ein Spektrum der von dem Material gestreuten Strahlung zu messen, **dadurch gekennzeichnet, dass** sie umfasst:

   - einen ersten Sensor (120), Hauptsensor genannt, der dazu geeignet ist, ein erstes Spektrum (150) der von dem Material (100) gestreuten Strahlung zu messen, und der so angeordnet ist, dass sein Betrachtungsfeld (130) das Strahlungsbündel (115) innerhalb des Materials (100) schneidet,

- zumindest einen Sensor (122, 124, 126), Nebensensor genannt, der dazu geeignet ist, zumindest ein weiteres Spektrum (152, 154, 156) der von dem Material (100) gestreuten Strahlung, sekundäres Spektrum genannt, zu messen,

- Einrichtungen zum Erstellen einer Matrix (X), Messungsmatrix genannt, und zwar ausgehend von den gemessenen Spektren,

- Computereinrichtungen zum Zerlegen der Messungsmatrix in zwei nicht negative Matrizes, nämlich in eine Matrix (A), Gewichtsmatrix genannt, von der jeder Term $a_{ij}$ den Anteil einer von dem Sensor i gemessenen, einfach oder mehrfach gestreuten Strahlung j darstellt, und in eine Matrix (S), Spektrenmatrix genannt, wobei letztere ein Spektrum geschätzter, mehrfach gestreuter Strahlung und ein Spektrum geschätzter, einfach gestreuter Strahlung umfasst; wobei diese Zerlegungseinrichtungen dazu geeignet sind, eine vorangehende Phase des Initialisierens der Gewichtsmatrix (A) und der Spektrenmatrix (S) auszuführen.

10. Vorrichtung nach Anspruch 9, **dadurch gekennzeichnet, dass** zumindest einer der Nebensensoren (124) so angeordnet ist, dass sein Betrachtungsfeld (134) das Strahlungsbündel (115) innerhalb des Materials (100) nicht schneidet.

11. Vorrichtung nach einem der vorangehenden Ansprüche, **dadurch gekennzeichnet, dass** zumindest zwei der Sensoren (120, 122, 126, 128) mit einem identischen Betrachtungsraumwinkel kollimiert sind.

12. Vorrichtung nach einem der vorangehenden Ansprüche, **dadurch gekennzeichnet, dass** zumindest einer der Sensoren (120, 122, 124, 126) kollimiert ist und dass die Quelle einfallender Strahlung (110) kollimiert ist, wobei die Raumwinkel des Strahlungsbündels und des Betrachtungsfeldes des Sensors identisch sind.

13. Vorrichtung nach einem der vorangehenden Ansprüche, **dadurch gekennzeichnet, dass** die Quelle (110) eine Röntgen- oder Gammastrahlungsquelle ist.

14. Vorrichtung nach einem der vorangehenden Ansprüche, **dadurch gekennzeichnet, dass** der Hauptsensor und die Nebensensoren (120, 122, 126, 128) dazu vorgesehen sind, eine rückgestreute Strahlung zu messen.

15. Computerprogramm mit einer Folge von Anweisungen, die bei Ausführung auf einem Mikroprozessor dazu geeignet sind, ein Verfahren nach einem der Ansprüche 1 bis 8 durchzuführen.

## Claims

1. A method of processing spectra of radiation scattered by a material (100) in order to obtain a spectrum of primary radiation scattered by said material, in which the material (100) is exposed to an incident irradiation beam (115) emitted by a radiation source (110) and at least one spectrum of radiation scattered by the material is measured, **characterized in that** it comprises:

- measuring a spectrum (150) of primary radiation scattered by the material (100) by means of a detector (120), referred to as main detector, arranged so that an observation field (130) of the main detector intersects the incident irradiation beam (115) inside the material (100),
- measuring at least one other spectrum (152, 154, 156) of radiation scattered by the material (100), referred to as secondary spectrum, by means of at least one detector (122, 124, 126) referred to as secondary detector,
- constituting a matrix (X), referred to as measurements matrix, from previously measured spectra,
- then performing decomposition of said measurements matrix into two non-negative matrices, which are a matrix (A) referred to as weights matrix, of which each term $a_{ij}$ represents the proportion of primary or multiple scattered radiation j measured by the detector i and a matrix (S), referred to as spectra matrix, the latter comprising an estimated multiple scattered radiation spectrum and an estimated primary scattered radiation spectrum, this decomposition being preceded by a step of initializing the weights matrix (A) and the spectra matrix (S).

2. A method according to claim 1, **characterized in that** at least one secondary detector (124) is arranged so that its observation field (134) does not intersect the irradiation beam (115) within the material (100).

3. A method according to claim 1 or claim 2, **characterized in that** the weights matrix (A) is initialized by extrapolation of data pre-established for a plurality of reference materials (Fig. 7 and 8).

4. A method according to claim 3, **characterized in that** the extrapolation is made essentially by using a density estimation as extrapolation variable.

**5.** A method according to one of the preceding claims, **characterized in that** the spectra matrix (S) is initialized with primary and multiple scattered radiation spectra (Fig. 5 and 6) simulated for a reference material exposed to said irradiation beam (115).

**6.** A method according to one of the preceding claims, **characterized in that** it comprises an iterative process of which each step comprises such a decomposition into non-negative matrices.

**7.** A method according to the preceding claim, **characterized in that** said iterative process is continued until a convergence criterion, concerning a proximity between the measurements matrix (X) and a product of non-negative matrices (AS), is met.

**8.** A method according to one of the preceding claims, **characterized in that** at least two of the detectors (120, 122, 124, 126) used are collimated and disposed, along the central axis of their observation field, at a same distance from a surface (105) of the material exposed to the irradiation beam (115).

**9.** A device for processing scattered radiation spectra in order to obtain a spectrum of primary radiation scattered by a material (100), comprising a source (110) of radiation suitable for emitting an incident irradiation beam (115) towards the material (100), and at least one detector suitable for measuring a spectrum of radiation scattered by the material (100), **characterized in that** it comprises:

- a first detector (120), referred to as main detector, suitable for measuring a spectrum (150) of primary radiation scattered by the material (100), and arranged so that its observation field (130) intersects the irradiation beam (115) inside the material (100),
- at least one detector (122, 124, 126) referred to as secondary detector, suitable for measuring at least one other spectrum (152, 154, 156), referred to as secondary spectrum, of radiation scattered by the material (100),
- means for constituting a matrix (X) referred to as measurements matrix, from the measured spectra,
- computer means for performing decomposition of said measurements matrix into two non-negative matrices, which are a matrix (A) referred to as weights matrix, of which each term $a_{ij}$ represents the proportion of primary or multiple scattered radiation j measured by the detector i and a matrix (S), referred to as spectra matrix, the latter comprising an estimated multiple scattered radiation spectrum and an estimated primary scattered radiation spectrum, these decomposition means being suitable for executing a prior phase of initializing the weights matrix (A) and the spectra matrix (S).

**10.** A device according to claim 9, **characterized in that** at least one of the secondary detectors (124) is arranged so that its observation field (134) does not intersect the irradiation beam inside the material (100).

**11.** A device according to one of the preceding claims, **characterized in that** at least two of the detectors (120, 122, 126, 128) are collimated with an identical solid observation angle.

**12.** A device according to one of the preceding claims, **characterized in that** at least one of the detectors (120, 122, 124, 126) is collimated and **in that** the incident radiation source (110) is collimated, the solid angles of the irradiation beam and of the observation field of said detector being identical.

**13.** A device according to one of the preceding claims, **characterized in that** the source (110) of irradiation is an X ray or gamma ray source.

**14.** A device according to one of the preceding claims, **characterized in that** the main and secondary detectors (120, 122, 126, 128) are arranged to measure back-scattered radiation.

**15.** A computer program comprising a series of instructions adapted, when they are executed by a microprocessor, to implement a method according to one of claims 1 to 8.

Fig. 1

Fig. 2

Fig. 3

Fig. 4

Fig. 5

Fig. 6

Fig. 7

Fig. 8

Fig. 9

Fig. 10

Fig. 11

Fig. 12

Fig. 13

Fig. 14

Fig. 15

Fig. 16

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2007007247 A
- EP 1566771 A1
- US 6320933 B

**Littérature non-brevet citée dans la description**

- **LEE, D. ; SEUNG, H.S.** Algorithms for Non-negative Matrix Factorization. *Adv. Neural Info. Proc. Syst.,* 2001, vol. 13, 556-562